(19) Europäisches Patentamt
European Patent Office
Office européen des brevets

(11) Veröffentlichungsnummer: **0 026 473**
**B1**

(12) # EUROPÄISCHE PATENTSCHRIFT

(45) Veröffentlichungstag der Patentschrift: **25.05.83**

(51) Int. Cl.³: **A 61 K 7/13**

(21) Anmeldenummer: **80105820.7**

(22) Anmeldetag: **25.09.80**

(54) **Mittel zur oxidativen Färbung von Haaren.**

(30) Priorität: **28.09.79 DE 2939303**

(43) Veröffentlichungstag der Anmeldung: **08.04.81 Patentblatt 81/14**

(45) Bekanntmachung des Hinweises auf die Patenterteilung: **25.05.83 Patentblatt 83/21**

(84) Benannte Vertragsstaaten: **DE GB IT SE**

(56) Entgegenhaltungen:

**DE-A-2 836 276**
**BEILSTEINS HANDBUCH DER ORGANISCHEN CHEMIE, 4. Auflage, 3. Ergänzungswerk, Band 10, 1971, Springer Verlag Berlin, DE Seite 1456, Formel VI; Seite 1457**

(73) Patentinhaber: **Wella Aktiengesellschaft, Berliner Allee 65, D-6100 Darmstadt (DE)**

(72) Erfinder: **Bachmann, Heinrich, Gässli 300, CH-1711 Giffers (CH)**
Erfinder: **Portmann, Plato, Prof. Dr., Cité-Jardins, CH-1700 Fribourg (CH)**

**EP 0 026 473 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Mittel zur oxidativen Färbung von Haaren

Zur Färbung von Haaren haben sogenannte Oxidationsfarbstoffe auf Grund ihrer Licht- und Waschechtheit eine wesentliche Bedeutung erlangt. Zur Erzeugung der Oxidationsfarbstoffe werden als Farbstoffvorstufen bestimmte, zur oxidativen Kupplung befähigte aromatische Verbindungen auf das Haar aufgebracht. Diese dringen teilweise in das Haar ein und werden dort entweder durch Luftsauerstoff oder insbesondere durch Zusatz chemischer Oxidationsmittel wie Hydrogenperoxid zum gewünschten Farbstoff oxidiert.

Als Farbstoffvorstufen dienen hauptsächlich Derivate von Diamino- oder Hydroxyamino-Verbindungen des Benzols, Naphthalins, Pyridins, Pyrimidins, Pyrazolons, Indols und Chinolins. Diese sind teilweise aus physiologischen Gründen nicht unbedenklich.

Ebenfalls bekannt als Farbstoffvorstufen sind Diphenol- und Naphthol-Derivate. Jedoch sind mit den bisher als Farbstoffvorstufen bekannten Vertretern dieser Substanzklassen keine intensiven Haarfärbungen erzielbar.

Weiterhin ist seit der Kenntnis der Bildung des natürlichen Haarpigmentes auch die Verwendung von Tyrosin, Dopa (Dopa = L-β-(3,4-Dihydroxy-phenyl)-alanin) und Dihydroxyindolen zum Färben von Haaren beschrieben worden. Mittel auf der Basis dieser, aus physiologischer Sicht für eine Verwendung als Farbstoffvorstufen wohl günstigsten Verbindungen konnten sich aus unterschiedlichen Gründen in der Praxis nicht durchsetzen.

Aus der deutschen Offenlegungsschrift 2 836 276 ist es bekannt, bestimmte 2,5-Dihydroxyphenyl-alkansäuren bzw. ihre Salze als Antioxidans in Haarfärbemitteln auf der Basis bekannter Oxidationsfarbstoffe zu verwenden. Über eine färberische Wirksamkeit dieser 2,5-Dihydroxyphenylalkansäuren wird in der genannten Offenlegungsschrift nicht berichtet.

An Oxidationsfarbstoffe, die zur Färbung menschlicher Haare Verwendung finden, sind zahlreiche besondere Anforderungen gestellt. So müssen sie in toxikologischer und dermatologischer Hinsicht unbedenklich sein und die Erzielung von Färbungen in der gewünschten Intensität ermöglichen. Ferner ist es erforderlich, daß durch Kombination von solchen als Farbstoffvorstufen geeigneten Verbindungen eine breite Palette verschiedener Farbnuancen erzeugt werden kann. Außerdem wird für die erzielbaren Haarfärbungen eine gute Licht-, Dauerwell-, Säure- und Reibechtheit gefordert. Auf jeden Fall aber müssen solche Haarfärbungen ohne Einwirkung von Licht, Reibung und chemischen Mitteln über einen Zeitraum von mindestens 4 bis 6 Wochen stabil bleiben.

Der Vielzahl der gestellten Anforderungen können die zur Zeit in Oxidationshaarfärbemitteln als Farbstoffvorstufen verwendeten Verbindungen jedoch nicht völlig zufriedenstellend genügen.

Es bestand daher die Aufgabe, Mittel zum oxidativen Färben von Haaren auf der Basis von solchen als Farbstoffvorstufen dienenden Verbindungen zu erstellen, welche in physiologischer Hinsicht gegenüber den bekannten als Farbstoffvorstufen eingesetzten Verbindungen günstigere Eigenschaften besitzen. Darüber hinaus sollten sie, allein oder in Kombination mit anderen als Farbstoffvorstufen bekannten Verbindungen, gute Trageechtheiten sowie genügende Intensität der Färbungen aufweisen.

Erfindungsgegenstand ist daher ein Mittel zur oxidativen Färbung von Haaren, enthaltend Wasser und/oder Alkohol sowie in Haarfärbemitteln übliche Zusätze, dadurch gekennzeichnet, daß es als Farbstoffvorstufe 0,1 bis 6,0 Gew.-% mindestens einer Verbindung der allgemeinen Formel

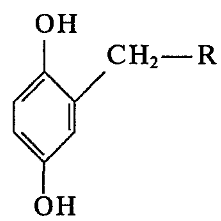

(I)

worin R die Bedeutung COOR', $CONH_2$, CONHR', $CONR'_2$, $CONH-NH_2$ oder CONH-OH hat und R' einen Alkylrest mit 1 bis 5 Kohlenstoffatomen darstellt, enthält.

Beispiele für geeignete, in den erfindungsgemäßen Haarfärbemitteln als Farbstoffvorstufen enthaltene Verbindungen der oben angegebenen allgemeinen Formel (I) sind

Homogentisinsäure-amid,
Homogentisinsäure-dimethylamid,
Homogentisinsäure-methylester,
Homogentisinsäure-ethylester,
Homogentisinsäure-isopropylester und
Homogentisin-hydroxamsäure (2,5-Dioxyphenylacethydroxamsäure).

Die Herstellung der Homogentisinsäurederivate gemäß Formel I ist zum wesentlichen Teil in der Literatur beschrieben. Soweit die Herstellung bestimmter Derivate nicht beschrieben ist, können diese ohne weiteres in analoger Weise nach den beschriebenen Herstellungsverfahren erhalten werden. So ist beispielsweise die Herstellung der Alkylester der Homogentisinsäure in der Literatur Beilstein, Handbuch d. Org. Chemie, Bd. 10, 1971, S. 1457 für den Methyl- und Ethylester beschrieben. Die Synthese von Homogentisinsäureamid und Homogentisinsäurealkylamiden kann zum Beispiel der Literatur K. Krohn, Tetrahedron Letters No. 52, S. 4667—4668 entnommen werden. Übliche Synthesewege zur Darstellung von Estern, Amiden, Hydraziden und Hydroxamsäuren aus den entsprechenden Säuren, Säurehalogeniden oder Estern, die auf die Homogentisinsäure übertragen werden können, sind weiterhin z. B. aus der Literatur Organikum, 9. Auflage, VEB Deutscher Verlag der Wissenschaften Berlin, 1970, S. 419–420, 441 und 449–459 beschrieben. Als weitere Literatur sei genannt: H. Beyer, Lehrbuch der Organischen Chemie, 15./16. Auflage, 1968, S. 211–212.

Darüber hinaus können die Haarfärbemittel der vorliegenden Anmeldung als Farbstoffvorstufen insbesondere natürlich vorkommende, aber auch andere geeignete, aromatische Verbindungen, die mindestens eine Hydroxygruppe im Molkül aufweisen und zusätzlich ein Stickstoffatom oder mehrere Stickstoffatome im Molekül haben können, enthalten.

Beispiele für solche Verbindungen sind.

a) aromatische Verbindungen mit mindestens einer Hydroxygruppe im Molekül wie Orcin, Hydrochinon, Pyrogallol, Hydroxyhydrochinon, Protocatechualdehyd, Thymol, Guajacol, Arbutin, Brenzcatechin, Juglon, Lawson, Vanillin, Pulvinsäure, Hydroxycumarin, Flavonoide und Grevelline, Derivate der Salicylsäure, der Kaffeesäure, der Chlorogensäure sowie ferner
b) aromatische Verbindungen mit mindestens einer Hydroxygruppe und zusätzlich mindestens einem Stickstoffatom im Molekül wie Dopa, Tyrosin, Kynurensäure und Xanthurensäure sowie die Derivate der Anthranilsäure, der Nicotinsäure, der Isonicotinsäure, der Picolinsäure, der Pyrrole, der Pyrimidine, der Purine, der Betalainsäuren, des Kynurenins und der Ommatine.

Diese Verbindungen können in einem Verhältnis von 0,1 bis 1 Mol, bezogen auf 1 Mol der als Farbstoffvorstufen enthaltenen Verbindungen der allgemeinen Formel (I), in den hier beschriebenen Mitteln eingesetzt werden.

Die Gesamtmenge der in den erfindungsgemäßen Haarfärbemitteln enthaltenen, als Farbstoffvorstufe dienenden Verbindungen beträgt zweckmäßigerweise etwa 0,1 bis 6 Gew.-%, vorzugsweise 1 bis 4 Gew.-%.

Außerdem ist es möglich, daß die Haarfärbemittel noch zusätzlich übliche direktziehende Haarfarbstoffe enthalten.

Zur Erhöhung der Farbtiefe der Haarfärbungen können in den Haarfärbemitteln gemäß dieser Anmeldung darüber hinaus natürlich vorkommende Aminosäuren, ihre Ester mit niederen Alkoholen und/oder ihre Amide sowie Mono- oder Dialkylamide, wobei die Alkyl-Gruppe am Amid-Stickstoff 1 bis 5 Kohlenstoffatome aufweist, enthalten sein.

Beispiele hierfür sind die Aminosäuren Glycin, Alanin, Prolin, Hydroxyprolin, Serin, Cystein, Histidin und Tryptophan, ihre Ester mit niederen Alkoholen sowie ihre Amide bzw. Mono- oder Dialkylamide, wobei die Alkylgruppe am Amid-Stickstoff 1 bis 5 Kohlenstoffatome aufweist.

Die Aminosäuren und ihre Derivate können dabei in den Mitteln insbesondere in einem Verhältnis von 0,05 bis 1 Mol, bezogen auf 1 Mol der darin als Farbstoffvorstufen vorhandenen Verbindungen, enthalten sein.

Schließlich können in den Haarfärbemitteln noch weitere übliche Zusätze, beispielsweise Antioxidantien wie Ascorbinsäure oder Natriumsulfit, Alkalisierungsmittel wie Alkalihydroxide, Ammonium- bzw. Alkalicarbonat und Ammonium- bzw. Alkalihydrogencarbonat, organische Säuren wie z. B. Essigsäure, Milchsäure und Zitronensäure, Lösungsmittel, Parfüm, Quellmittel, Netzmittel, Emulgatoren, Verdicker, Pflegestoffe und andere vorhanden sein.

Die Zubereitungsform kann die einer Lösung, vorzugsweise einer Creme, eines Gels oder einer Emulsion sein. Ihre Zusammensetzung stellt eine Mischung der Farbstoffkomponenten mit den für solche Zubereitungen üblichen Bestandteilen dar. Als übliche Bestandteile von Cremes, Gelen oder Emulsionen kommen beispielsweise Netzmittel oder Emulgatoren aus den Klassen der anionischen, kationischen oder nichtionogenen oberflächenaktiven Substanzen wie Fettalkoholsulfate, Alkylsulfonate, Alkylbenzolsulfonate, Alkyltrimethylammoniumsalze, oxethylierte Fettalkohole, oxethylierte Nonylphenole, Fettsäurealkanolamide, ferner Verdicker wie höhere Fettalkohole, Stärke, Zellulosederivate, Paraffinöl und Fettsäuren sowie außerdem Pflegestoffe wie Lanolinderivate, Cholesterin und Pantothensäure in Betracht. Die erwähnten Bestandteile werden in den für solche Zwecke üblichen Mengen verwendet, z. B. die Netzmittel und Emulgatoren in Konzentrationen von etwa 0,5 bis 30 Gewichtsprozent, während die Verdicker in einer Menge von etwa 0,1 bis 25 Gewichtsprozent in den Zubereitungen enthalten sein können.

Je nach Zusammensetzung können die erfindungsgemäßen Haarfärbemittel schwach sauer, neutral oder alkalisch reagieren. Insbesondere weisen sie einen pH-Wert im alkalischen Bereich zwischen 7,5 und 11 auf, wobei die Einstellung vorzugsweise mit Ammoniak oder Ammoniumhydrogencarbonat erfolgt. Es können aber auch organische Amine, zum Beispiel Monoethanolamin oder Triethanolamin, Verwendung finden.

Ihre Anwendung kann je nach dem pH-Wert des erfindungsgemäßen Mittels, nach bekannten Verfahren A) in einer, B) in zwei oder C) in drei Stufen erfolgen.

A) Bei dem einstufigen Verfahren vermischt man die Haarfärbemittel kurz vor dem Gebrauch mit einem Oxidationsmittel und trägt das Gemisch auf das Haar auf. Als Oxidationsmittel zur Entwicklung der Haarfärbung kommen hauptsächlich Peroxidsulfate oder Hydrogenperoxid, letztes beispielsweise als 3%ige wäßrige Lösung, bzw. dessen Additionsverbindungen an Harnstoff, Melamin oder Natriumborat in Betracht. Die Anwendungstemperaturen der Haarfärbemittel liegen im Bereich von 20 bis 50° C. Nach einer Einwirkungszeit von etwa

20 bis 40 Minuten, vorzugsweise etwa 30 Minuten, wird das Haar mit der wäßrigen Lösung einer schwachen organischen Säure, wie z. B. der Zitronensäure oder Weinsäure, die gegebenenfalls noch EDTA*) enthält, gespült und schließlich mit Wasser nachgespült.

*) EDTA = Ethylendiamintetraacetat

Dieses Verfahren ist insbesondere für die Anwendung von solchen erfindungsgemäßen Haarfärbemitteln geeignet, die auf einen pH-Wert von etwa 9 bis 10 eingestellt sind.

B) Das zweistufige Verfahren ist für die Anwendung der hier beschriebenen Haarfärbemittel insbesondere dann geeignet, wenn diese einen pH-Wert zwischen 7 und 8 aufweisen. Hierbei wird in der ersten Stufe das Haarfärbemittel auf das Haar aufgetragen und bei einer Temperatur von 20 bis 50° C etwa 10 bis 20 Minuten lang einwirken gelassen. Danach bringt man in der zweiten Stufe eine etwa 1 bis 8%ige alkalische Lösung eines geeigneten Oxidationsmittels auf das Haar auf, wobei sich der pH-Wert des auf das Haar einwirkenden Gemisches etwa auf 9,5 erhöht. Diese Lösung wird ebenfalls etwa 10 bis 20 Minuten lang einwirken gelassen. Anschließend wird das Haar mit der wäßrigen Lösung einer schwachen organischen Säure, wie zum Beispiel der Zitronensäure oder Weinsäure, die gegebenenfalls noch EDTA enthält, gespült und schließlich mit Wasser nachgespült.

C) Das dreistufige Verfahren ist für solche erfindungsgemäßen Haarfärbemittel geeignet, welche einen pH-Wert im schwach sauren Bereich aufweisen. Hierbei wird in der ersten Verfahrensstufe das Haarfärbemittel auf das Haar aufgetragen und etwa 10 Minuten lang bei einer Temperatur von etwa 35 bis 45° C gelassen. In der zweiten Verfahrensstufe bringt man ein Oxidationsmittel, beispielsweise eine 10%ige Ammoniumperoxodisulfat-Suspension, auf das Haar auf und läßt diese ebenfalls etwa 10 Minuten lang bei der gleichen Temperatur einwirken. In der dritten Verfahrensstufe schließlich verteilt man die wäßrige Lösung eines Alkalisierungsmittels, beispielsweise eine 9%ige Ammoniaklösung, auf dem Haar und läßt sie etwa 15 Minuten lang bei der gleichen Temperatur einwirken. Sodann wird das Haar zunächst mit der wäßrigen Lösung einer schwachen organischen Säure, wie z. B. Zitronensäure oder Weinsäure, die gegebenenfalls noch EDTA enthält, gespült und darauf mit Wasser nachgespült.

Die nachstehenden Beispiele sollen den Gegenstand der Erfindung näher erläutern.

## Beispiele

### Beispiel 1

| | |
|---|---|
| 3,00 g | Homogentisinsäureamid |
| 0,80 g | Orcin |
| 0,03 g | Glycinkupfer |
| 7,50 g | Guanidin-hydrochlorid |
| 2,00 g | Ammoniumhydrogencarbonat |
| 1,00 g | Hydroxyethylcellulose, mittelviskos |
| 20,00 g | Ethanol |
| 65,67 g | Wasser |
| 100,00 g | |

Diese Haarfärbelösung, die einen pH-Wert von pH = 7,8 aufweist, wird auf gebleichte menschliche Haare aufgetragen und 15 Minuten lang bei einer Temperatur von 37 bis 40° C einwirken gelassen. Anschließend werden 50 ml einer wäßrigen Lösung von 5 Gew.-% Ammoniak und 1,5 Gew.-% Hydrogenperoxid auf das Haar aufgebracht. Diese Lösung wird ebenfalls 15 Minuten lang bei der gleichen Temperatur einwirken gelassen. Danach wird das Haar zunächst mit einer 10%igen Zitronensäurelösung, die zusätzlich 0,05 Gew.% EDTA enthält, gespült und schließlich mit Wasser nachgespült. Das Haar hat eine natürlich wirkende braune Färbung erhalten.

### Beispiel 2

| | |
|---|---|
| 3,00 g | Homogentisinsäure-methylester |
| 0,80 g | Guajacol |
| 0,03 g | Glycinkupfer |
| 7,50 g | Guanidin-hydrochlorid |
| 2,00 g | Ammoniumhydrogencarbonat |
| 1,00 g | Hydroxyethylcellulose, mittelviskos |
| 20,00 g | Ethanol |
| 65,67 g | Wasser |
| 100,00 g | |

Die Anwendung dieser Haarfärbelösung, die einen pH-Wert von pH = 7,8 aufweist, erfolgt wie in Beispiel 1 beschrieben. Dunkelblonde, natürliche menschliche Haare haben nach der Behandlung eine neutrale, natürlich wirkende braune Färbung erhalten.

Beispiel 3

| | |
|---|---|
| 3,00 g | Homogentisinsäure-ethylester |
| 1,00 g | Orcin |
| 0,50 g | Glycin-methylester |
| 0,03 g | Glycinkupfer |
| 7,50 g | Guanidin-hydrochlorid |
| 2,00 g | Ammoniumhydrogencarbonat |
| 1,00 g | Hydroxyethylcellulose, mittelviskos |
| 20,00 g | Ethanol |
| 64 97 g | Wasser |
| 100,00 g | |

Diese Haarfärbelösung, die einen pH-Wert von pH = 7,8 aufweist, wird auf nicht vorbehandelte graue menschliche Haare aufgetragen und 15 Minuten lang bei einer Temperatur von 37 bis 40°C einwirken gelassen. Anschließend werden 50 ml einer wäßrigen Lösung von 5 Gew.-% Ammoniak und 1,5 Gew.-% Hydrogenperoxid auf das Haar aufgebracht. Diese Lösung wird ebenfalls 15 Minuten lang bei der gleichen Temperatur einwirken gelassen. Danach wird das Haar zunächst mit einer 10%igen Zitronensäurelösung, die zusätzlich 0,05 Gew.-% EDTA enthält, gespült und schließlich mit Wasser nachgespült. Das Haar hat eine rotbraune Färbung erhalten.

Beispiel 4

| | |
|---|---|
| 2,00 g | Homogentisinsäure-isopropylester |
| 1,00 g | Homogentisin-hydroxamsäure |
| 0,50 g | Guanidin |
| 0,50 g | Prolin-methylester |
| 0,03 g | Glycinkupfer |
| 7,50 g | Guanidin-hydrochlorid |
| 2,00 g | Ammoniumhydrogencarbonat |
| 1,00 g | Hydroxyethylcellulose, mittelviskos |
| 20,00 g | Ethanol |
| 65,47 g | Wasser |
| 100,00 g | |

Diese Haarfärbung, die einen pH-Wert von pH = 7,8 aufweist, wird auf gebleichte menschliche Haare aufgetragen und 15 Minuten lang bei einer Temperatur von 37 bis 40°C einwirken gelassen. Anschließend werden 50 ml einer wäßrigen Lösung von 5 Gew.-% Ammoniak und 1,5 Gew.-% Hydrogenperoxid auf das Haar aufgebracht. Diese Lösung wird ebenfalls 15 Minuten lang bei der gleichen Temperatur einwirken gelassen. Danach wird das Haar zunächst mit einer 10%igen Zitronensäurelösung, die zusätzlich 0,05 Gew.-% EDTA enthält, gespült und schließlich mit Wasser nachgespült. Das Haar hat eine rotstichige dunkelbraune Färbung erhalten.

Beispiel 5

| | |
|---|---|
| 3,00 g | Homogentisinsäure-dimethylamid |
| 1,00 g | Guajacol |
| 0,50 g | Glycinamid |
| 0,03 g | Glycinkupfer |
| 9,50 g | Guanidin-hydrochlorid |
| 1,00 g | Ammoniumhydrogencarbonat |
| 2,50 g | Ammoniak |
| 0,35 g | Natriumhydrogensulfit |
| 1,00 g | Hydroxyethylcellulose, mittelviskos |
| 40,00 g | Ethanol |
| 41,12 g | Wasser |
| 100,00 g | |

Man vermischt diese Haarfärbelösung kurz vor dem Gebrauch mit 20 ml Hydrogenperoxidlösung (3%ig), trägt das Gemisch auf gebleichte menschliche Haare auf und läßt es 30 Minuten lang bei einer Temperatur von 37 bis 40°C einwirken. Danach wird das Haar zunächst mit einer 10%igen Zitronensäurelösung, die zusätzlich 0,05 Gew.-% EDTA enthält, gespült und schließlich mit Wasser nachgespült. Das Haar hat eine natürliche Braunfärbung ohne Rotstich erhalten.

Beispiel 6

| | |
|---|---|
| 2,00 g | Homogentisinsäure-isopropylester |
| 1,00 g | Homogentisinsäure-dimethylamid |
| 1,00 g | Glycinamid |
| 0,50 g | Prolin-methylester |
| 0,03 g | Glycinkupfer |
| 9,50 g | Guanidin-hydrochlorid |
| 1,00 g | Hydroxyethylcellulose, mittelviskos |
| 1,00 g | Zitronensäure |
| 30,00 g | Ethanol |
| 53,97 g | Wasser |
| 100,00 g | |

Man bringt dieses gelförmige Haarfärbemittel auf gebleichte menschliche Haare auf und läßt es 10 Minuten lang bei einer Temperatur von 37 bis 40°C einwirken. Anschließend werden 25 ml einer 10%igen wäßrigen Ammoniumperoxodisulfat-Suspension gleichmäßig auf dem Haar verteilt und 10 Minuten lang bei der gleichen Temperatur einwirken gelassen. Danach bringt man 25 ml einer 9%igen Ammoniaklösung auf das Haar auf und läßt diese weitere 15 Minuten lang bei der gleichen Temperatur einwirken. Sodann wird das Haar zunächst mit einer 10%igen Zitronensäurelösung, die zusätzlich 0,05 Gew.-% EDTA enthält, gespült und schließlich mit Wasser nachgespült. Das Haar hat eine braune Färbung erhalten.

Alle in der vorliegenden Anmeldung angegebenen Prozentzahlen stellen Gewichtsprozente dar.

**Patentansprüche**

1. Mittel zur oxidativen Färbung von Haaren, enthaltend Wasser und/oder Alkohol sowie in Haarfärbemitteln übliche Zusätze, dadurch gekennzeichnet, daß es als Farbstoffvorstufe 0,1 bis 6,0 Gew.-% mindestens einer Verbindung der allgemeinen Formel

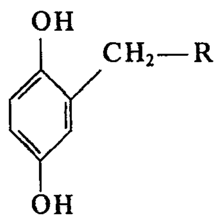

(I)

worin R die Bedeutung COOR', $CONH_2$, CONHR', $CONR_2'$, $CONH-NH_2$ oder CONH-OH hat und R' einen Alkylrest mit 1 bis 5 Kohlenstoffatomen darstellt, enthält.

2. Mittel nach Anspruch 1, dadurch gekennzeichnet, daß es als Farbstoffvorstufe mindestens eine der Verbindungen Homogentisinsäureamid, Homogentisinsäure-dimethylamid, Homogentisinsäure-methylester, Homogentisinsäure-ethylester, Homogentisinsäure-isopropylester und Homogentisinhydroxamsäure enthält.

3. Mittel nach Anspruch 1, dadurch gekennzeichnet, daß es als Farbstoffvorstufe Verbindungen gemäß Formel (I) in einer Konzentration von insgesamt 1 bis 4 Gew.-% enthält.

4. Mittel nach Anspruch 1 bis 3, dadurch gekennzeichnet, daß es zusätzlich andere als Farbstoffvorstufen geeignete Verbindungen sowie direktziehende Farbstoffe enthält.

5. Mittel nach Anspruch 4, dadurch gekennzeichnet daß es als Farbstoffvorstufe mindestens eine Verbindung enthält, die ausgewählt ist aus

a) den aromatischen Verbindungen mit mindestens einer Hydroxygruppe im Molekül Orcin, Hydrochinon, Pyrogallol, Hydroxyhydrochinon, Protocatechualdehyd, Thymol, Guajacol, Arbutin, Brenzkatechin, Juglon, Lawson, Vanillin, Pulvinsäure, Hydroxycumarin, Flavonoiden und Grevellinen, Derivaten der Salicylsäure, der Kaffeesäure, der Chlorogensäure, sowie ferner

b) den aromatischen Verbindungen mit mindestens einer Hydroxygruppe und zusätzlich mindestens einem Stickstoffatom im Molekül Dopa, Tyrosin, Kynurensäure und Xanthurensäure sowie den Derivaten der Anthranilsäure, der Nicotinsäure, der Isonicotinsäure, der Picolinsäure, der Pyrrole, der Pyrimidine, der Purine, der Betalainsäuren, des Kynurenins und der Ommatine.

6. Mittel nach Anspruch 1 bis 5, dadurch gekennzeichnet, daß die Gesamtmenge der als Farbstoffvorstufen enthaltenen Verbindungen 0,1 bis 6 Gew.-%, vorzugsweise 1 bis 4 Gew.-%, beträgt.

7. Mittel nach Anspruch 1 bis 6, dadurch gekennzeichnet, daß es zusätzlich natürlich vorkommende Aminosäuren, deren Ester mit niederen Alkoholen und/oder deren Amide sowie Mono- oder Dialkylamide, wobei die Alkylgruppe am Amid-Stickstoff bis 5 Kohlenstoffatome aufweist, enthält.

**Claims**

1. Composition for the oxidative dyeing of hair with a content of water and/or alcohol and additives usual in hairdyes, characterized in that it contains as dye intermediate 0.1 to 6.0 weight percent of at least one compound of the general formula

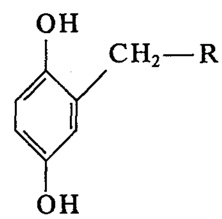

(I)

wherein R signifies COOR', $CONH_2$, CONHR', $CONR_2'$, $CONH-NH_2$ or CONH-OH and R' represents an alkyl group of 1 to 5 carbon atoms.

2. Composition according to claim 1, characterized in that it contains as dye intermediate at least one of the compounds homogentisic acid amide, homogentisic acid dimethylamide, homogentisic acid methylester, homogentisic acid ethylester, homogentisic acid isopropylester and homogentisic hydroxamic acid.

3. Composition according to claim 1, characterized in that in contains the dye intermediates corresponding to formula (I) in a total concentration of 1 to 4 weight percent.

4. Composition according to any of claims 1 to 3, characterized in that it additionally contains other compounds suitable as dye intermediates and also direct dyes.

5. Composition according to claim 4, characterized in that it contains as dye intermediate at least one compound selected from

a) the aromatic compounds with a minimum of one hydroxy group in the molecule i. e. orcin, hydroquinone, pyrogallol, hydroxyhydroquinone, protocatechualdehyde, thymol, guajacol, arbutin, pyrocatechol, juglone, lawsone, vanillin, pulvinic acid, hydroxy coumarin, flavonoids, and grevillines, derivatives of salicyclic acid, caffeic

acid, chlorogenic acid and, furthermore,

b) the aromatic compounds with a minimum of one hydroxy group and, additionally, a minimum of one nitrogen atom in the molecule, i. e. dopa, tyrosine, kynurenic acid and xanthurenic acid, as well as the derivatives of anthranilic acid, nicotinic acid, isonicotinic acid, picolinic acid, of pyrrols, pyrimidines, purines, betalainic acids, kynurenin and of ommatins.

6. Composition according to any of the claims 1 to 5, characterized in that the total quantity of compounds contained therein as dye intermediates amounts to 0.1 to 6.0 weight percent, preferably to 1 to 4 weight percent.

7. Composition according to any of the claims 1 to 6, characterized in that it additionally contains amino acids which occur in nature, the esters thereof with lower alcohols and/or their amides, as well as mono- or dialkyl amides, with the alkyl group bonded therein to the nitrogen of the amide has 1 to 5 carbon atoms.

**Revendications**

1. Composition pour la teinture par oxydation des cheveux, contenant de l'eau et/ou de l'alcool ainsi que des adjuvants habituels dans les compositions pour la teinture des cheveux, caractérisée en ce qu'elle contient en tant que progéniteur du colorant de 0,1 à 6,0% en poids au moins d'un composé dont la formule générale est:

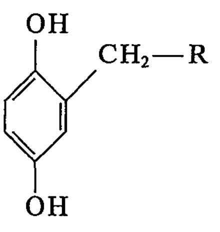

(I)

où R représente COOR', $CONH_2$, CONHR', $CONR_2'$, CONH-$NH_2$ ou CONH-OH et R' est un radical alkyle contenant de 1 à 5 atomes de carbone.

2. Composition selon la revendication 1, caractérisée en ce qu'elle contient en tant que progéniteur du colorant au moins l'un des composés acide homogentisinique-amide, acide homogentisinique-diméthylamide, acide homogentisinique-méthylester, acide homogentisinique-éthylester, acide homogentisinique-isopropyléther et acide homogentisinique-hydroxamique.

3. Composition selon la revendication 1, caractérisée en ce qu'elle comprend en tant que progéniteur de colorant des composés selon la formule (I) selon une concentration d'ensemble de 1 à 4% en poids.

4. Composition selon une des revendications 1 à 3, caractérisée en ce qu'elle contient en outre d'autres composés pour constituer des progéniteurs de colorants ainsi que des colorants à fixation directe.

5. Composition selon la revendication 4, caractérisée en ce qu'elle contient en tant que progéniteur de colorant au moins un composé qui est choisi parmi:

a) les composés aromatiques comprenant au moins un groupe hydroxy dans la molécule orcine, hydroquinone, pyrogallol,hydroxyhydroquinone,protocatéchualdéhyde, thymol, gaïacol, arbutine, cathéchol, juglon Lawson, vanilline, acide pulvinique, hydroxycoumarine, des flavonoïdes et des grevellines, des dérivés de l'acide salicylique, de l'acide caféïque, de l'acide chlorogénique, ainsi qu'en outre:

b) les composés aromatiques comprenant au moins un groupe hydroxy et en plus au moins un atome d'azote dans la molécule dopa, tyrosine, acide cynurénique et acide xanthurénique ainsi que des dérivés de l'acide anthranilique, de l'acide nicotinique, de l'acide isonicotinique, de l'acide picolinique, du pyrrole, de la pyrimidine, de la purine, des acides bétalainiques, de la cynurénine et de l'ommatine.

6. Composition selon l'une des revendications 1 à 5, caractérisée en ce que la quantité totale des composés contenus en tant que progéniteurs des colorants est de 0,1 à 6% en poids et de préférence de 1 à 4% en poids.

7. Composition selon l'une des revendications 1 à 6, caractérisée en ce qu'elle comprend en plus des acides aminés d'origine naturelle, dont les esters avec des alcools faibles et/ou dont l'amide ainsi que la mono ou la dialkylamide, le groupe alkyle comprenant sur l'azote-amide de 1 à 5 atomes de carbone.